# EUROPEAN PATENT SPECIFICATION

(11) **EP 0 650 721 B1**
(45) Date of publication and mention of the grant of the patent: **13.09.2000**
(21) Application number: 94110184.2
(22) Date of filing: 30.06.1994
(51) Int. Cl.: A61K 9/107, A61K 9/48, A61K 38/13

(54) **Cyclosporin soft capsule**
Cyclosporin enthaltende Weichkapsel
Capsule molle à base de cyclosporine

(30) Priority: 01.07.1993 KR 9312291
(43) Date of publication of application: 03.05.1995
(73) Proprietor: HANMI PHARM. IND. CO., LTD., Whasung-gun, Kyunggi-do (KR)
(72) Inventor: Woo, Jong Soo, Suwon-shi, Kyunggi-do (KR)
(74) Representative: Dost, Wolfgang, Dr.rer.nat., Dipl.-Chem.

(56) References cited:
- WO-A-87/06463
- WO-A-94/05312

## Description

### Background of the Invention

### 1. Field of the invention

The present invention relates to a soft capsule composition containing a stable microemulsion concentrate suitable for the preparation of cyclosporin-containing soft capsules. More particularly, the present invention relates to a microemulsion concentrate containing cyclosporin as an active ingredient, dimethylisosorbide as a co-surfactant, an oil component and a surfactant, which is suitable for formulation into soft capsules and to a soft capsule composition containing said microemulsion concentrate.

### 2. Background art

Cyclosporin is a specific macromolecular (molecular weight 1202.64) cyclic peptide compound consisting of 11 amino acids, which has useful pharmacological activities, particularly immunosuppressive activity and anti-inflammatory activity. Therefore, cyclosporin has been used for suppression of immunological responses native to the living body, which are caused by tissue and organ transplantation, for example, transplantation of the heart, lung, liver, kidney, pancreas, bone marrow, skin and cornea, and particularly the transplantation of foreign tissues and organs. In addition, cyclosporin is useful for the suppression of autoimmune diseases and inflammatory diseases such as arthritis, etc.

Cyclosporin is highly lipophilic and hydrophobic with a solubility in water at 25^{o}C being 16 to 23mg of cyclosporin per liter of water. The bioavailability of cyclosporin is also extremely low due to its low water-solubility. On the other hand, cyclosporin is well dissolved in an organic solvent such as methanol, ethanol, acetone, ether, chloroform and the like, due to its high lipophilic property.

As with other conventional drugs which are sparingly soluble in water, cyclosporin is very difficult to formulate into a preparation for oral administration due to its low water solubility and bioavailability. Further, since the bioavailability of cyclosporin may be greately influenced by the condition of each individual patient, it is very difficult to provide an effective therapeutic effect.

Moreover, it is very important to provide a uniform dosage amount and appropriate bioavailability since counterbalancing the efficacy of cyclosporin are its considerable toxic side effects such as nephrotoxicity, hepatotoxicity, among others. Accordingly, numerous studies have been extensively and widely conducted to find a preparation suitable for the effective oral administration of cyclosporin.

The prior art preparations suitable for oral administration of sparingly water-soluble cyclosporin are usually formulated in the form of a microemulsion. In preparing the liquid microemulsion formulation, cyclosporin should be combined with a surfactant, an oil and a cosurfactant. One method using this combination is taught in U.S. Patent No. 4,388,307 which issued on June 14, 1983. This patent discloses a liquid formulation of cyclosporin using ethanol as a cosurfactant. Thus, cyclosporin is combined with a carrier consisting of ethanol as a cosurfactant, a vegetable oil and a transesterification product of a natural vegetable oil triglyceride and a polyalkylene glycol as a surfactant to form the liquid formulation. However, the resulting liquid formulation is administered as an aqueous dilution making it very difficult to properly formulate the preparation to provide a uniform dosage for oral administration.

In order to alleviate the requirement of diluting the cyclosporin liquid composition in water prior to oral administration, a liquid composition in the form of a microemulsion concentrate has been formulated into a soft capsule preparation, which is presently commercially available as Sandimmun^{R} (trademark). In this preparation the cyclosporin soft capsule contains a large amount of ethanol as a cosurfactant due to the solubility requirements of cyclosporin. However, since ethanol, which has a low boiling point, permeates the gelatin membrane of the capsule to volatilize even at normal temperature, the constitutional ratio of the contents in soft capsules varies during storage. The reduced ethanol content results in a significant difference in the bioavailability of cyclosporin and making the problem worse, the cyclosporin crystallizes when the soft capsules are stored at low temperature.

WO 94/05312 discloses in claims 1 to 3 and in example 1 a soft gelatine capsule comprising cyclosporin A, an amount of 1,4:3,6-dianhydro-2,5-di-O-methyl-D-glucitole, a plant oil and a surfactant for medical treatment.

In an effort to prevent the volatilization of ethanol from the soft capsule preparations during storage and distribution, the soft capsule preparations are wrapped in a packing material, such as an aluminum film foam package. However, such specific packaging does not completely maintain the uniform composition of the wrapped capsule. It has been demonstrated through experiments that although the cyclosporin soft capsule is wrapped up in aluminum film foam package, the ethanol content is lowered to 7.9% from the initial level of 10.8% after a period of one week. This results in a great difference in bioavailability and may contribute to the increase of its price.

To solve the above-mentioned disadvantages which accompany the use of ethanol as a cosurfactant, a method using a non-ethanol component as a cosurfactant has been proposed. Korean Laid-open Patent Publication No. 90-4348 (April 12, 1990) discloses a pharmaceutical composition in the form of a microemulsion concentrate containing a non-ethanol component. The non-ethanol components include pharmaceutically acceptable C₁₋₅ alkyl or tetrahydrofurfuryl di- or partial-ether of low molecular mono- or poly-oxy-alkanediol, for example, diethyleneglycol monoethyl ether [e.g. Transcutol] or tetrahydrofurfuryl alcohol polyethylene glycol ether [e.g. Glycofurol], or 1,2-propyleneglycol as a cosurfactant. However, the above non-ethanol cosurfactants are glycols which contain the -OH group in their structures. It has now been identified that the OH group-containing glycol creates problems in the formulation of soft capsules because its strong absorption property is sufficient to absorb the moisture from the atmosphere and also because it is highly permeable to the gelatin film of the soft capsule.

Thus, the present inventors have studied numerous additives, including various solvents, in an effort to find a cosurfactant capable of providing a microemulsion concentrate suitable for the formulation of cyclosporin into a soft capsule preparation. As a result, a certain pharmaceutically acceptable solvent, dimethylisosorbide [Trade name: Arlasove^{R} DMI, available from ICI Speciality Chemicals] has been found as a suitable solvent for this purpose to complete the present invention.

Therefore, it is an object of the present invention to provide a microemulsion concentrate containing dimethylisosorbide as a cosurfactant, which is suitable for formulation into soft capsules for oral administration.

It is a further object of the present invention to provide a microemulsion concentrate suitable for formulation into soft capsules, which contains cyclosporin as an active ingredient, dimethylisosorbide as a cosurfactant, an oil component and a surfactant.

Further, it is another object of the present invention to provide a soft gelatin capsule composition according to the present invention which is highly storage stable such that there is little variation of the composition over time.

The foregoing has outlined some of the more pertinent objects of the present invention. These objects should be construed to be merely illustrative of some of the more pertinent features and applications of the invention. Many other beneficial results can be obtained by applying the disclosed invention in a different manner or modifying the invention within the scope of the disclosure. Accordingly, other objects and a more thorough understanding of the invention may be had by referring to the disclosure of invention and the drawings, in addition to the scope of the invention defined by the claims.

In accordance with the above, an oral microemulsion composition as defined in the claims is provided.

### BRIEF DESCRIPTION OF THE DRAWINGS

For a thorough understanding of the nature and objects of the invention, reference should be had to the following detailed description taken in connection with the accompanying drawings in which:

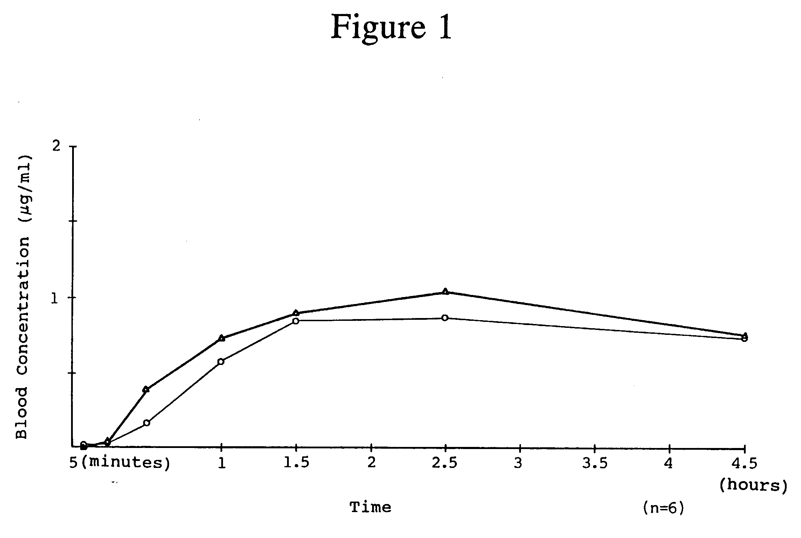

Figure 1 is a graph showing the change in blood concentration over time after cyclosporin microemulsion concentrate containing dimethylisosorbide as a cosurfactant according to Example 1 ( △-△ ) and cyclosporin microemulsion concentrate containing ethanol as a cosurfactant ( ○-○ ) are orally administered, and

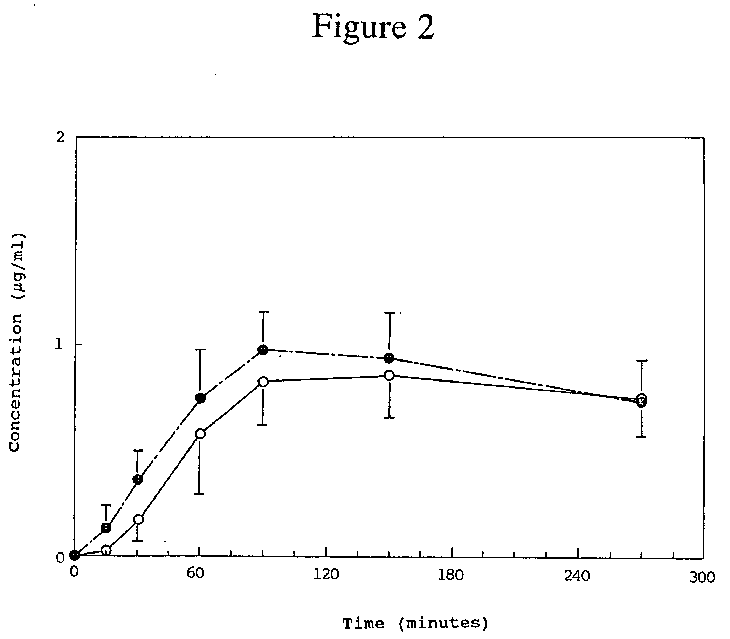

Figure 2 is a graph showing the change in blood concentration over time after cyclosporin microemulsion concentrate containing dimethylisosorbide as a cosurfactant according to Example 6 ( ●-● ) and cyclosporin emulsion concentrate containing ethanol as a cosurfactant ( ○-○ ) (Sandimmun^{R}) are orally administered to rabbit.

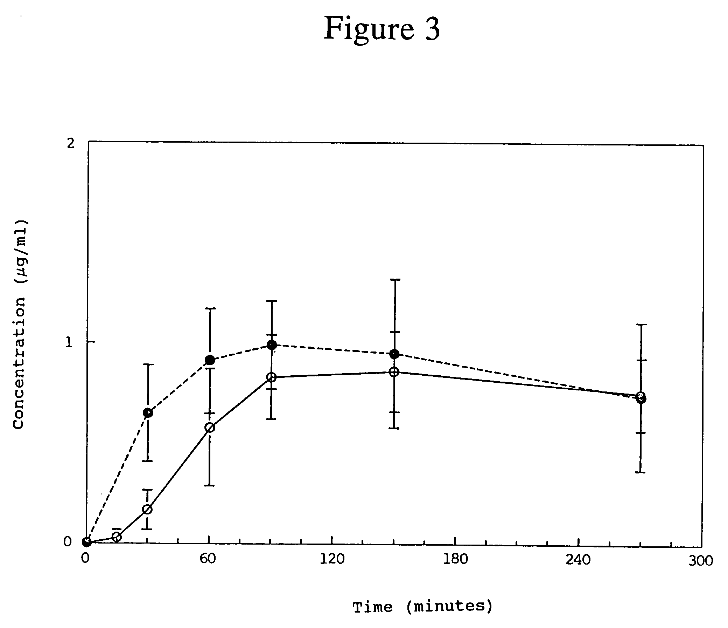

Figure 3 is a graph showing the change in blood concentration over time after cyclosporin microemulsion concentrate containing dimethylisosorbide as a cosurfactant according to Example 7 ( ●-● ) and cyclosporin emulsion concentrate containing ethanol as a cosurfactant ( ○-○ ) (Sandimmun^{R}) are orally administered to rabbit.

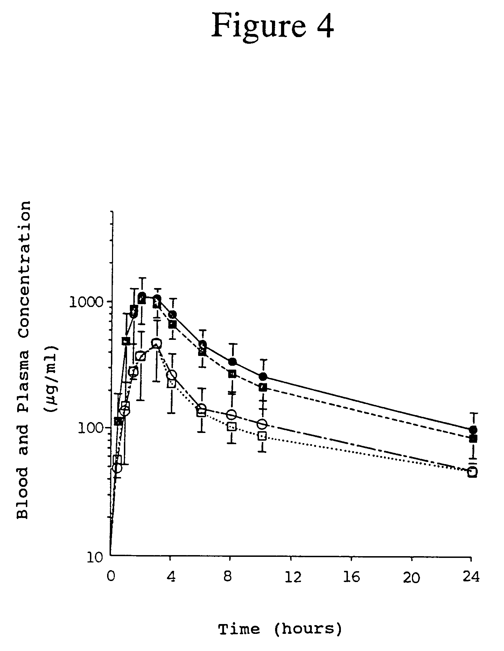

Figure 4 is a graph showing the concentration of cyclosporin in the whole blood and plasma after the composition of Example 6 containing dimethylisosorbide as a cosurfactant according to the present invention and the commercialized product Sandimmun^{R} are orally administered to human [ ●-● blood concentration of Sandimmun^{R}; ■-■ blood concentration of the composition of Example 6 according to the present invention; ○-○ plasma concentration of Sandimmun^{R}; □-□ plasma concentration of the composition of Example 6 according to the present invention].

### DISCLOSURE OF INVENTION

In one aspect, the present invention relates to a microemulsion concentrate comprising cyclosporin as an active ingredient, dimethylisosorbide as a cosurfactant, an oil component and a surfactant which is suitable for the formulation of soft capsules for oral administration, as defined in the claims.

Cyclosporin, the first essential component is the pharmaceutically active ingredient in the microemulsion concentrate according to the present invention. Cyclosporin is a cyclic peptide compound having useful immuno-suppressive activity and antiinflammatory activity as described above and known in the art. Although cyclosporin A, B, C, D, G and the like can be used as the cyclosporin component in the present invention, cyclosporin A is most preferred since its clinical effectiveness and pharmacological properties are well established in the art.

The dimethylisosorbide cosurfactant, which is the second essential component in the microemulsion concentrate according to the present invention, is a compound represented by the following structural formula. Dimethylisosorbide has been used as a skin permeation stimulator in topical pharmaceutical compositions. The chemical name of dimethylisosorbide is 1,4:3,6-dianhydro-2,5-dimethyl-D-glucitol.

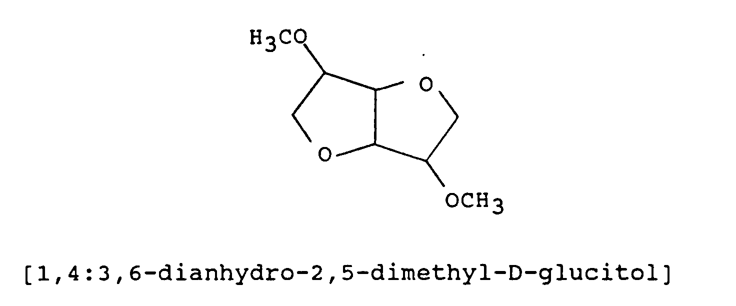

Since dimethylisosorbide has a high boiling point, 234^{o}C, it does not volatilize even at high temperature such as the temperature necessary for manufacturing soft capsules. In addition, dimethylisosorbide does not contain any hydroxy groups, -OH, and therefore, its hygroscopic property is very low and it does not permeate the gelatin membrane of the soft capsule. Most importantly, cyclosporin dissolves well in dimethylisosorbide which contributes to the formulation of a suitable microemulsion as defined above.

Accordingly, the use of dimethylisosorbide as a cosurfactant in the microemulsion concentrate of the present invention provides certain advantages. That is, when the microemulsion concentrate is formulated into a soft capsule, a change in the composition of the concentrate does not appear during storage and the contents of the components contained therein are substantially uniformly maintained so that the uniformity of the composition content can be assured over a greater time period than ethanol based compositions.

In the microemulsion concentrate of the present invention, dimethylisosorbide is used in the ratio of 1 to 5 parts by weight, preferably 2 to 4 parts by weight, and most preferably 4 parts by weight, per 1 part by weight of cyclosporin.

The third component used in the microemulsion concentrate according to the present invention is an oil namely refined fish oil. The oil component suitable for use in the present invention may further include any animal or vegetable oils which are pharmaceutically acceptable. Thus, neutral animal oils and vegetable oils, for example, castor oil or corn oil, particularly animal oil rich in unsaturated fatty acids having an iodine value of 100 to 200, for example, refined fish oil and vegetable oil having the hydroxyl value of 150, for example, castor oil, are preferably used. Among such oils, the most preferred oil is refined fish oil. Refined fish oil is ideally fit for the absorption of cyclosporin since it contains EPA (eicosapentaenoic acid), a highly unsaturated fatty acid, and DHA (docosahexaenoic acid) in the amount of 18%, or more, and 12%, or more, respectively, having a total unsaturated fatty acid content of 30%, or more, which allows for the absorption of cyclosporin.

The oil component according to the present invention may include only refined fish oil or a mixture of refined fish oil and an oil(s) selected from the above-mentioned oils. When a mixture of two or more oil components is used, it is preferred that at least one animal oil and at least one vegetable oil be present in said mixture.

In addition, if required, in the oil component the appopriate amount of saturated fatty acids, for example, caprilic acid/capric triglyceride [Trademark: Miglyol 812; Dynamit Nobel Chemikalien] can be included in order to control the absorption of cyclosporin. When such saturated fatty acid is added to the oil component, it can modulate the absorption pattern of cyclosporin to reduce the occurrence of typical side effects involved in cyclosporin preparations.

The fourth essential component used in the microemulsion concentrate according to the present invention is a surfactant. The suitable surfactants for use in the present invention include any of pharmaceutically acceptable surfactant capable of stably emulsifying the lipophilic portion of the composition comprising the cyclosporin-containing oil component and the hydrophilic part comprising the cosurfactant in water to form a stable microemulsion. The most preferred surfactants according to the present invention include polyoxyethylene glycolated natural or hydrogenated vegetable oils, transesterification reaction products of natural vegetable oil triglyceride and polyalkylene polyol, sorbitan fatty acid esters, polyoxyethylene-sorbitan fatty acid esters, polyoxyethylene alcohols, etc., such as the reaction products of castor oil and ethylene oxide, which are commercially available under the trade mark CREMOPHOR (BASF), the esterification reaction products of 2 moles of natural vegetable oil and 1 mole of polyethylene glycol, which are commercially available under the trade mark LABRAFIL (Etablissement Gattefosse), the mono- and tri-lauryl, palmityl, stearyl and oleyl esters which are commercially available under the trade mark TWEEN (Lippo Chemicals), the sorbitan fatty acid esters which are commercially available under the trade mark SPAN (Lippo Chemicals) and the polyoxyethylene alcohols which are commercially available under the trade mark BRIJ (BASF). Among these surfactans, Labrafil M 1944 CS (apricot kernel oil PEG-6 ester) and Tweens having the HLB Value of 14.0 to 15.0 are the most preferred. The surfactant may include any one of the above-mentioned surfactants alone or, preferably, in a combination of one or more surfactants selected from the above surfactants.

In the microemulsion concentrate according to the present invention, four essential components are present preferably in the ratio of cyclosporin:cosurfactant:oil component:surfactant= 1:1-5:1-5:2-10, and more preferably in the ratio of cyclosporin: cosurfactant:oil component:surfactant=1:2-4:2-5:2-7 by weight. The most preferable microemulsion concentrate according to the present invention consists of cyclosporin A, dimethylisosorbide, Labrafil and refined fish oil in the ratio of 1:4:3:2.7 by weight. In addition to this composition, the composition illustrated in the following examples can be mentioned as the further preferable compositions according to the present invention.

For oral administration, the microemulsion concentrate having the above-mentioned composition, according to the present invention, can be formulated into the form of a soft capsule. In formulating the soft capsule, the capsule preparation can further contain, if necessary, pharmaceutically acceptable adjuvants, excipients and additives which are conventionally utilized in the preparation of soft capsules, in addition to the above microemulsion concentrate. Such additives include, for example, lecithin, viscosity regulator, perfume (e.g. peppermint oil, etc.), antioxidant (e.g. tocopherol, etc.), preservative (e.g. parabens, etc.), coloring agent, glyerin, sorbitol, gelatine, etc.

The soft capsule preparation according to the present invention can be prepared according to conventional methods for the preparation of soft capsules. For example, cyclosporin is first dissolved in dimethylisosorbide while gently warming at the temperature of approximately 60^{o}C. The oil component and the surfactant are then added to the resulting mixture and the whole mixture is uniformly mixed. The resulting microemulsion concentrate is then introduced into a machine for preparing soft capsules, with or without the above-mentioned pharmaceutically acceptable additives conventionally utilized in preparation of soft capsules, to prepare the desired suitable cyclosporin soft capsule. The soft capsule composition thus prepared according to the present invention exhibits a blood level of cyclosporin comparable to that of the prior art ethanol-containing soft capsule preparation when they are administered by mouth, i.e. orally. Further the soft capsule composition according to the present invention is stably maintained without any change over a prolonged storage perior. Accordingly, it is readily apparent that the present invention provides a significant improvement in the field of preparation of the cyclosporin soft capsules.

The present invention will be more specifically illustrated by the following examples. However, it should be understood that the present invention is not limited by these examples in any manner.

### Example 1

2.5g of cyclosporin A was added to 4.5g of dimethylisosorbide (Arlasolve^{R} DMI). The mixture was warmed to about 60^{o}C with stirring to dissove cyclosporin. To the resulting solution were added 7.5g of Labrafil M 1944CS (apricot kernel oil PEG-6 ester) and 11.5g of refined fish oil and the mixture was sufficiently stirred until the homogeneous microemulsion concentrate was formed. The microemulsion concentrate thus obtained was introduced into a machine for preparation of soft capsules, which is adjusted so that 0.26g of the microemulsion concentrate is injected into one soft capsule, to prepare the soft gelatin capsules containing the cyclosporin microemulsion concentrate within gelatin sheets.

### Example 2

The soft gelatin capsules having the following compositions were prepared according to the same procedure as Example 1 except that the amount of dimethylisosorbide as a cosurfactant is varied.

| 2-A. | |
|---|---|
| Component | Content(mg/Cap.) |
| Cyclosporin | 25 |
| Dimethylisosorbide(Arlasolve^{R} DMI) | 25 |
| Labrafil M 1944 CS | 75 |
| Refined fish oil | 115 |
| | Total 240mg |

| 2-B. | |
|---|---|
| Component | Content(mg/Cap.) |
| Cyclosporin | 25 |
| Dimethylisosorbide(Arlasolve^{R} DMI) | 65 |
| Labrafil M 1944 CS | 75 |
| Refined fish oil | 115 |
| | Total 280mg |

### Example 3

The soft gelatin capsules having the following compositions were prepared according to the same procedure as Example 1 except that as the surfactant the mixture of Labrafil and another surfactant is used instead of Labrafil alone.

| 3-A. | |
|---|---|
| Component | Content(mg/Cap.) |
| Cyclosporin | 25 |
| Dimethylisosorbide(Arlasolve^{R} DMI) | 45 |
| Labrafil M 1944 CS | 75 |
| Tween 80 | 5 |
| Refined fish oil | 115 |
| | Total 265mg |

| 3-B. | |
|---|---|
| Component | Content(mg/Cap.) |
| Cyclosporin | 25 |
| Dimethylisosorbide(Arlasolve^{R} DMI) | 45 |
| Labrafil M 1944 CS | 75 |
| Cremophor EL | 5 |
| Refined fish oil | 115 |
| | Total 265mg |

| 3-C. | |
|---|---|
| Component | Content(mg/Cap.) |
| Cyclosporin | 25 |
| Dimethylisosorbide(Arlasolve^{R} DMI) | 45 |
| Labrafil M 1944 CS | 75 |
| Span 20 | 5 |
| Refined fish oil | 115 |
| | Total 265mg |

| 3-D. | |
|---|---|
| Component | Content(mg/Cap.) |
| Cyclosporin | 25 |
| Dimethylisosorbide(Arlasolve^{R} DMI) | 45 |
| Labrafil M 1944 CS | 75 |
| Brij 35 | 5 |
| Refined fish oil | 115 |
| | Total 265mg |

### Example 4

The soft gelatin capsules having the following compositions were prepared according to the same procedure as Example 1 except that the oil component and its content are changed.

| 4-A. | |
|---|---|
| Component | Content(mg/Cap.) |
| Cyclosporin | 25 |
| Dimethylisosorbide(Arlasolve^{R} DMI) | 45 |
| Labrafil M 1944 CS | 75 |
| Refined fish oil | 55 |
| | Total 200mg |

| 4-B. | |
|---|---|
| Component | Content(mg/Cap.) |
| Cyclosporin | 25 |
| Dimethylisosorbide(Arlasolve^{R} DMI) | 45 |
| Labrafil M 1944 CS | 75 |
| Refined fish oil | 115 |
| | Total 320mg |

| 4-C. | |
|---|---|
| Component | Content(mg/Cap.) |
| Cyclosporin | 25 |
| Dimethylisosorbide(Arlasolve^{R} DMI) | 45 |
| Labrafil M 1944 CS | 75 |
| Refined fish oil | 115 |
| Caprilic acid/Capric triglyceride | 40 |
| | Total 300mg |

| 4-D. | |
|---|---|
| Component | Content(mg/Cap.) |
| Cyclosporin | 25 |
| Dimethylisosorbide(Arlasolve^{R} DMI) | 45 |
| Labrafil M 1944 CS | 80 |
| Refined fish oil | 69 |
| Castor oil | 46 |
| | Total 265mg |

### Example 5

The soft gelatin capsule having the following composition was prepared according to the same procedure as Example 1, except that peppermint oil (perfume) and tocopherol (antioxidant) are additionally added to the microemulsion concentrate before it is formulated into the soft capsule.

| Component | Content(mg/Cap.) |
|---|---|
| Cyclosporin | 25 |
| Dimethylisosorbide(Arlasolve^{R} DMI) | 45 |
| Labrafil M 1944 CS | 75 |
| Refined fish oil | 115 |
| Peppermint oil | 20 |
| Tocopherol | 5 |
| | Total 285mg |

### Example 6

The soft gelatin capsule having the following composition was prepared according to the same procedure as Example 1.

| Component | Content(mg/Cap.) |
|---|---|
| Cyclosporin | 25 |
| Dimethylisosorbide(Arlasolve^{R} DMI) | 50 |
| Labrafil M 1944 CS | 75 |
| Refined fish oil | 115 |
| Castor oil | 3.5 |
| d-α-Tocopherol | 1.5 |
| | Total 270mg |

### Example 7

The soft gelatin capsule having the following composition was prepared according to the same procedure as Example 1.

| Component | Content(mg/Cap.) |
|---|---|
| Cyclosporin | 25 |
| Dimethylisosorbide(Arlasolve^{R} DMI) | 100 |
| Labrafil M 1944 CS | 75 |
| Refined fish oil | 68.5 |
| d-α-Tocopherol | 1.5 |
| | Total 270mg |

### Example 8 (Comparative Example)

The membrane permeation property of dimethylisosorbide over the storage period at the room temperature was examined for the soft capsules prepared using dimethylisosorbide (Arlasolve^{R} DMI) as a cosurfactant according to the present invention and then compared with the membrane permeation properties of ethanol, propylene glycol, Transcutol (diethylene glycol monoethyl ether) and Glycofurol (tetrahydrofurfuryl alcohol polyethylene glycol ether). The content of the cosurfactant in each soft capsule preparation was analyzed by gas chromatography. The components and their constitutional ratios in the compositions used in this experiment are given in the following Table 1 and the experiment results are given in the following Table 2.

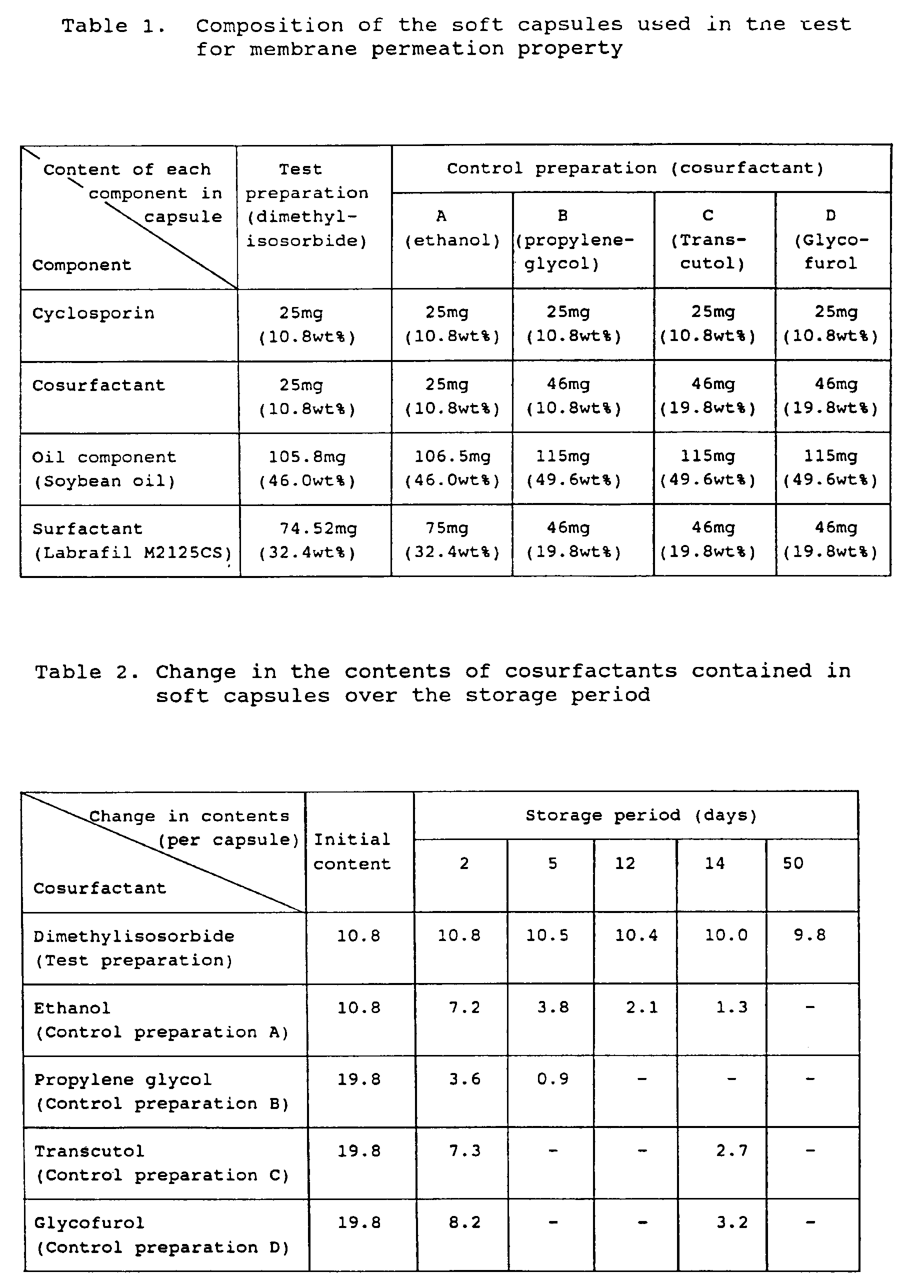

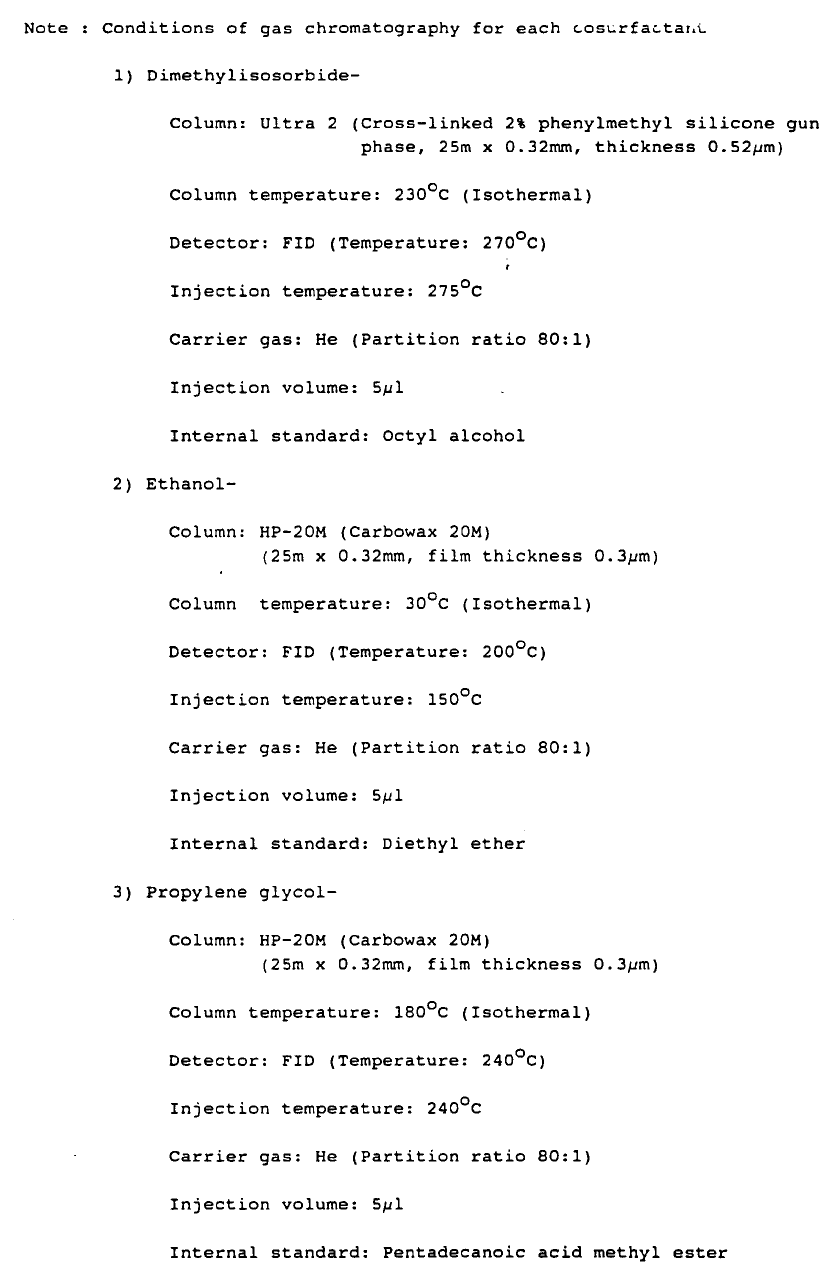

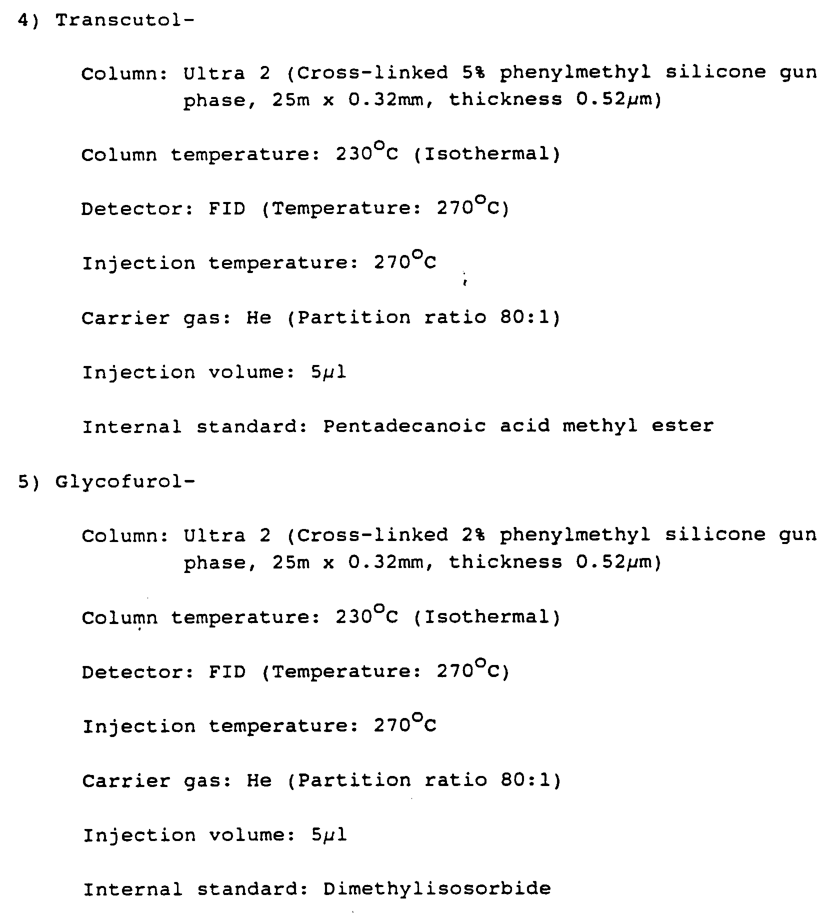

As can be seen from the results described in the above Table 2, dimethylisosorbide used as a cosurfactant according to the present invention does not change in its content even after 50 days while other cosurfactants used in the prior art were reduced by 50% of the initial content after only 2 days. Accordingly, it can be readily determined that dimethylisosorbide when used as a cosurfactant according to the present invention exhibits substantially no membrane permeation since the amount thereof does not significantly change in the composition and therefore is most suitable for use in the formulation of soft capsule preparations according to the present invention.

### Example 9

The bioavailability of the microemulsion using dimethylisosorbide as a cosurfactant according to the present invention was compared with the bioavailability of an ethanol-containing preparation according to the prior art to estimate the influence of dimethylisosorbide on the bioavailability of cyclosporin. In this experiment, rabbits were used as the experimental animal. The soft capsules as prepared in Example 1, Example 6 and Example 7 and the commercialized product SANDIMMUN^{R} using ethanol as a cosurfactant were used as the test preparations and the control preparation, respectively. In this experiment, both of the test preparations and the control preparation were administered in an amount of 300mg as cyclosporin per kg of rabbit. Rabbits were uniformly fed with the conventional rabbit solid feed composition for 4 days or more under the same condition in wire cages. When the oral preparations were administered, rabbits were fasted for 48 hours in a restraint cage made of wood, during which rabbits were allowed to freely take 10% dextrose solution. Levin's tube having a diameter of 5mm was interposed by the depth of 30cm through the esophagus after the surface of the Levin's tube was coated with vaseline in order to reduce friction. Each of the test preparations and the control preparation was emulsified with 50ml of water and then introduced into a syringe which is attached to the Levin's tube. Ear veins of rabbit were dilated using xylene and then blood was taken from each rabbit's ear vein at an interval of 5, 15, 30, 60, 90, 150 and 270 minutes by means of heparin-treated disposable syringe. To 1ml of blood thus obtained were added 0.5ml of aqueous saturated sodium chloride solution and 2ml of ether, and then the mixture was shaken for 5 minutes and centrifuged with 5000rpm for 10 minutes to separate the supernatant. 1ml of the supernatant was collected and then developed in an activated silica sep-pak^{R} (Waters). The developed sep-pak was washed with 5ml of n-hexane and eluated with 2ml of methanol. The eluate was evaporated to dryness in nitrogen gas under reduced pressure. The residue was analyzed by means of HPLC (High Performance Liquid Chromatography) [HPLC condition : Column µ-Bondapak^{R} C₁₈(Waters), Mobile phase CH₃CN : MeOH : H₂0 = 55 : 15 : 30, Detection 210nm, Flow rate 1.0ml/min., Column temperature 70^{o}C, Sensitivity 0.01 Aufs, Injection volume 100µl].

The results are illustrated at Figures 1, 2 and 3 for Examples 1, 6 and 7, respectively. As can be seen from Figures 1, 2 and 3, since the soft capsule preparation according to the present invention shows substantially the same blood level as that of the ethanol-containing soft capsule preparation according to the prior art, it is apparent that dimethylisosorbide used in the present invention exhibits a superior effect as to the stability of soft capsule formulation without influencing the bioavailability of cyclosporin.

### EXAMPLE 10

The bioavailability of the microemulsion using dimethylisosorbide as a cosurfactant according to the present invention was compared with the bioavailability of an ethanol-containing preparation according to the prior art to estimate the influence of dimethylisosorbide on the bioavailability of cyclosporin in human body. In this experiment, 16 healthy volunteeres aging 20 to 25 in average were involved and the soft capsules as prepared in Example 6 and the commercialized product SANDIMMUN^{R} using ethanol as a cosurfactant were used as the test preparation and the control preparation, respectively. In this experiment, both of the test preparation and the control preparation were administered in an amount of 400mg as cyclosporin per human body to examine the biological equivalency between two preparations. The test was conducted by dividing the 16 volunteeres into two groups as described in the following and then subjecting them to the cross experiment according to a Latin square cross over design. The interval between Period I and Period II was eight days.

| Subject | Period I | Period II |
|---|---|---|
| Group I | Control Preparation | Test Preparation |
| Group II | Test Preparation | Control Preparation |

Blood was taken from the subjects before and 0.5, 1, 1.5, 2, 3, 4, 6, 8, 10 and 24 hours after the administration of each preparation in an amount of 8ml each time and analyzed by means of a cyclosporin monoclonal analysis kit using monoclonal antibody to determine the concentration of cyclosporin in the whole blood and plasma. To 150µl of whole blood or plasma which was treated with heparin as an anticoagulant were added 50µl of a solubilization reagent and 300µl of a precipitation reagent for whole blood and then the mixture was agitated for 10 seconds to precipitate the protein and centrifuged with 9500xg for 5 minutes to obtain the supernatant which was then analyzed with TDX system.

The results are illustrated at Figure 4. As can be seen from Figure 4, since the soft capsule preparation according to the present invention shows substantially the same blood level as that of the ethanol-containing soft capsule preparation according to the prior art in human body, it is apparent that dimethylisosorbide used in the present invention has no effect on the bioavailability of cyclosporin.

Accordingly, the use of dimethylisosorbide as a cosurfactant in the cyclosporin-containing soft capsule according to the present invention effectively prolongs the shelf life of the capsules without adversly affecting bioavailability. Further, the cyclosporin pharmaceutical preparation according to the present invention overcomes a major problem in the soft capsules containing conventionally used cosurfactants, such as ethanol, propylene glycol, transcutol, glycofurol and the like. That is, the present invention alleviates membrane permeation of cosurfactant and the resulting change in the composition contained in the soft capsule which may lead to the crystallization of cyclosporin.

## Claims

1. An oral microemulsion composition for suppression of an immunological response comprising an immunosuppressive amount of cyclosporin and a sufficient amount of dimethylisosorbide, refined fish oil and surfactant to form a microemulsion suitable for oral administration, said cyclosporin and dimethylisosorbide being present in a ratio of 1:1-5 by weight.

2. The oral microemulsion composition of claim 1 further including a pharmaceutically acceptable adjuvant or excipient therefor.

3. The oral microemulsion composition of claim 1 or 2 wherein said cyclosporin is cyclosporin A.

4. The oral microemulsion composition of any of claims 1-3 wherein said composition may further comprise an oil selected from the group consisting of: an animal oil having an iodine value of 100 to 200, a vegetable oil having a hydroxyl value of 150 or more, and a mixture thereof.

5. The oral microemulsion composition of any of the preceding claims wherein said oil further includes a saturated fatty acid.

6. The oral microemulsion composition of claim 5 wherein said saturated fatty acid is selected from the group consisting of: caprilic acid and capric triglyceride, and a mixture thereof.

7. The oral microemulsion composition of any of the preceding claims wherein said surfactant is selected from the group consisting of: Labrafil M 1944CS, Tweens, Cremophor EL, Brij, Spans, and a mixture thereof.

8. The oral microemulsion composition of any of the preceding claims wherein said surfactant is selected from the group consisting of: Labrafil M 1944CS and Tweens.

9. The oral microemulsion composition of any of the preceding claim wherein said cyclosporin, said dimethylisosorbide, said oil and said surfactant are present in a ratio of 1:1-5:1-5:2-10, by weight.

10. The oral microemulsion composition of claim 9 wherein said cyclosporin, said dimethylisosorbide, said oil and said surfactant are present in a ratio of 1:2-4:2-5:2-7, by weight.

11. The oral microemulsion composition of any of the preceding claims formulated into a soft gelatin capsule.

## Patentansprüche

1. Orale Mikroemulsions-Zusammensetzung zum Unterdrücken einer immunologischen Reaktion, umfassend eine immunsuppressive Menge an Cyclosporin und eine ausreichende Menge an Dimethylisosorbid, gereinigtem Fischöl und einem oberflächenaktiven Mittel unter Bildung einer Mikroemulsion, die für eine orale Verabreichung geeignet ist, wobei das Cyclosporin und das Dimethylisosorbid in einem Verhältnis von 1:1-5 Gew.-% vorliegen.

2. Orale Mikroemulsions-Zusammensetzung nach Anspruch 1, die weiterhin ein pharmazeutisch verträgliches Adjuvans oder einen pharmazeutisch verträglichen Arzneimittelträger hierfür enthält.

3. Orale Mikroemulsions-Zusammensetzung nach Anspruch 1 oder 2, wobei das Cyclosporin Cyclosporin A ist.

4. Orale Mikroemulsions-Zusammensetzung nach einem der Ansprüche 1-3, wobei die Zusammensetzung weiterhin ein Öl, ausgewählt aus einem tierischen Öl mit einem Jodwert von 100 bis 200, einem Pflanzenöl mit einem Hydroxylwert von 150 oder mehr, und einem Gemisch davon, umfassen kann.

5. Orale Mikroemulsions-Zusammensetzung nach einem der vorangehenden Ansprüche, wobei das Öl weiterhin eine gesättigte Fettsäure enthält.

6. Orale Mikroemulsions-Zusammensetzung nach Anspruch 5, wobei die gesättigte Fettsäure ausgewählt ist aus n-Caprylsäure und n-Capryltriglycerid und einem Gemisch davon.

7. Orale Mikroemulsions-Zusammensetzung nach einem der vorangehenden Ansprüche, wobei das oberflächenaktive Mittel ausgewählt ist aus Labrafil M 1944CS, Tweens, Cremophor EL, Brij, Spans und einem Gemisch davon.

8. Orale Mikroemulsions-Zusammensetzung nach einem der vorangehenden Ansprüche, wobei das oberflächenaktive Mittel ausgewählt ist aus Labrafil M 1944CS und Tweens.

9. Orale Mikroemulsions-Zusammensetzung nach einem der vorangehenden Ansprüche, wobei das Cyclosporin, das Dimethylisosorbid, das Öl und das oberflächenaktive Mittel in einem Verhältnis von 1:1-5:1-5:2-10 Gew.-% vorliegen.

10. Orale Mikroemulsions-Zusammensetzung nach Anspruch 9, wobei das Cyclosporin, das Dimethylisosorbid, das Öl und das oberflächenaktive Mittel in einem Verhältnis von 1:2-4:2-5:2-7 Gew.-% vorliegen.

11. Orale Mikroemulsions-Zusammensetzung nach einem der vorangehenden Ansprüche, formuliert in einer Weichgelatinekapsel.

## Revendications

1. Composition de microémulsion orale pour la suppression d'une réponse immunologique comprenant une quantité immunosuppressive de cyclosporine et une quantité suffisante de diméthylisosorbide, d'huile de poisson raffinée et de tensioactif pour former une microémulsion convenant à l'administration orale, lesdits cyclosporine et diméthylisosorbide étant présents dans un rapport de 1 : 1-5 en poids.

2. Composition de microémulsion orale selon la revendication 1 contenant en outre un adjuvant ou excipient pharmaceutiquement acceptable pour celle-ci.

3. Composition de microémulsion orale selon la revendication 1 ou 2 dans laquelle ladite cyclosporine est la cyclosporine A.

4. Composition de microémulsion orale selon l'une quelconque des revendications 1 à 3 dans laquelle ladite composition peut contenir en outre une huile choisie dans le groupe constitué de : une huile animale ayant un indice d'iode de 100 à 200, une huile végétale ayant un indice d'hydroxyle de 150 ou plus, et un mélange de celles-ci.

5. Composition de microémulsion orale selon l'une quelconque des revendications précédentes dans laquelle ladite huile comprend en outre un acide gras saturé.

6. Composition de microémulsion orale selon la revendication 5 dans laquelle ledit acide gras saturé est choisi dans le groupe constitué de : acide caprylique et triglycéride caprique, et un mélange de ceux-ci.

7. Composition de microémulsion orale selon l'une quelconque des revendications précédentes dans laquelle ledit tensioactif est choisi dans le groupe constitué de : Labrafil M 1944CS, les Tweens, Cremophor EL, Brij, les Spans, et un mélange de ceux-ci.

8. Composition de microémulsion orale selon l'une quelconque des revendications précédentes dans laquelle ledit tensioactif est choisi dans le groupe constitué de : Labrafil M 1944CS et les Tweens.

9. Composition de microémulsion orale selon l'une quelconque des revendications précédentes dans laquelle ladite cyclosporine, ledit diméthylisosorbide, ladite huile et ledit tensioactif sont présents dans un rapport de 1 : 1-5 : 1-5 : 2-10, en poids.

10. Composition de microémulsion orale selon la revendication 9 dans laquelle ladite cyclosporine, ledit diméthylisosorbide, ladite huile et ledit tensioactif sont présents dans un rapport de 1 : 2-4 : 2-5 : 2-7, en poids.

11. Composition de microémulsion orale selon l'une quelconque des revendications précédentes formulée en une capsule de gélatine molle.
